Europäisches Patentamt

European Patent Office

Office européen des brevets

(11) Veröffentlichungsnummer: **0 152 945 B1**

# EUROPÄISCHE PATENTSCHRIFT

(45) Veröffentlichungstag der Patentschrift: **09.09.92**

(51) Int. Cl.⁵: **A61K 9/10**

(21) Anmeldenummer: **85101809.3**

(22) Anmeldetag: **20.02.85**

(54) Pharmazeutische Mehrkomponentensysteme und Verfahren zu ihrer Herstellung.

(30) Priorität: **23.02.84 DE 3406497**

(43) Veröffentlichungstag der Anmeldung:
**28.08.85 Patentblatt 85/35**

(45) Bekanntmachung des Hinweises auf die
Patenterteilung:
**09.09.92 Patentblatt 92/37**

(84) Benannte Vertragsstaaten:
**BE CH DE FR GB IT LI NL SE**

(56) Entgegenhaltungen:
**EP-A- 0 100 448**
**FR-A- 2 304 392**
**FR-A- 2 502 951**
**US-A- 4 073 943**
**US-A- 4 146 499**

**CHEMICAL ABSTRACTS, Band 92, Nr. 22, 2.
Juni 1980, Seite 333, Zusammenfassung Nr.
186335r, Columbus, Ohio, US; G. RIMLINGER:
"Study on emulsions. Part II. Mixed theory of
emulsions", & RELATA TECH. 1979, 11(19),
45-62**

**Pharm.Ind. 50 (1988), 370-375 und 1301-1306**

(73) Patentinhaber: **Müller, Bernd Willy Werner,
Prof. Dr.
Schlotfeldtsberg 14a
W-2302 Flintbek(DE)**

(72) Erfinder: **Müller, Bernd Willy Werner, Prof.Dr.
Schlotfeldtsberg 14a
W-2302 Flintbek(DE)**
Erfinder: **Franzky, Hans-Jürgen
von Coels-Strasse 1
W-5100 Aachen(DE)**
Erfinder: **Kölln, Claus-Jürgen
Eichofstrasse 9
W-2300 Kiel(DE)**

(74) Vertreter: **UEXKÜLL & STOLBERG Patentanwälte
Beselerstrasse 4
W-2000 Hamburg 52(DE)**

## Beschreibung

Die Erfindung betrifft flüssige, transparente, pharmazeutisch anwendbare Mehrkomponentensysteme.

Die therapeutische Wirksamkeit von Arzneimitteln wird maßgeblich durch die passive Verfügbarkeit, nämlich die Freisetzung aus dem applizierten System, sowie durch die biologische Verfügbarkeit, nämlich die Membranpassage des Wirkstoffes beeinflußt. Der jeweils langsamer ablaufende Schritt bestimmt die Geschwindigkeit der Aufnahme des Wirkstoffes durch den Organismus. Beispielsweise muß bei kutaner Applikation der Wirkstoff zunächst aus der Salbe freigesetzt werden und anschließend die Hornschicht der Haut durchdringen, bevor er durch eine Membran aufgenommen und weitertransportiert werden kann. Gewöhnlich ist die Passage durch die Hornschicht der langsamste und daher geschwindigkeitsbestimmende Schritt. Er kann durch die Wahl der Salbengrundlage, d.h. einer Wasser-in-Öl- (W/O) oder Öl-in-Wasser-Emulsion (O/W), oder durch Einarbeiten von Penetrationsvermittlern, sogenannten Gleitschienen, beschleunigt werden. Diese Möglichkeit ist jedoch insofern begrenzt, als bei Molekülen mit einem Molekulargewicht oberhalb 800 eine Penetrationsgeschwindigkeit von $10^{-4}$ $\mu g/cm^2 \cdot h$ praktisch nicht überschritten wird. Ferner wirkt die Basalmembran der Lederhaut aufgrund ihrer Ladung fixierend oder abstoßend auf alle geladenen Teilchen, so daß beispielsweise dissoziierte Stoffe diese Zellschicht nicht oder nur sehr eingeschränkt zu durchdringen vermögen. In ähnlicher Weise verhält sich die Membran im Rektum. Der aus der verabreichten Arzneiform freigesetzte, gelöste Wirkstoff muß zunächst die Glycocalix passieren, bevor er durch die Zellmembran aufgenommen werden kann. Ist hier beispielsweise eine Diffusion in die Zellschicht aufgrund eines zu hohen Molekulargewichts des Wirkstoffes nicht möglich, so kann keine wesentliche Absorption stattfinden. Durch den Zusatz von Gleitschienen kann auch hier eine begrenzte Aufnahme erreicht werden; aus toxikologischen Gründen ist dies jedoch problematisch.

Hochdisperse, flüssige, transparente, einphasige Systeme sind aus der chemischen Technologie seit langem bekannt und in neuerer Zeit als Möglichkeit zur Gewinnung des Restöls aus den Gesteinsschichten der Erde beschrieben worden, vgl. D.O. Shah, "Surface Phenomena in Enhanced Oil Recovery", Plenum Press, New York 1981. Die pharmazeutische Anwendung derartiger Systeme scheiterte bisher jedoch an der Toxizität der verwendeten ionischen Tenside. Mit nicht-ionischen Tensiden wie Polyethylenglykol-(PEG)-sorbitanfettsäureestern in Kombination mit Fettsäuren oder Fettsäureestern bilden sich bei Zimmertemperatur lediglich Makroemulsionen. Erst bei höheren Temperaturen werden im Phasenumkehrbereich Mikroemulsionen erhalten, vgl. Shinoda, J. Colloid Interf. Sci. 32, 647 (1970). Die Ausnutzung dieses Phänomens wurde erstmals durch Ziegenmeyer und Führer, "Acta Pharm. Techn." 26, 273 (1980) beschrieben; die Autoren brachten Tetracyclinhydrochlorid durch die Haut, indem sie die Phasenumkehrtemperatur des Makroemulsionssystems, d.h. das Umkippen der Emulsion von einer O/W- zu einer W/O-Emulsion auf die Hauttemperatur, nämlich 32°C, einstellten. Derartige Systeme sind jedoch aufgrund ihrer physikalischen Instabilität pharmazeutisch nicht nutzbar. Ferner konnte die Verbesserung der Penetration durch die Haut lediglich in einem in vitro Versuch mit einer therapeutisch zu niedrigen Wirkstoffkonzentration gezeigt werden.

Ferner beschreibt S. Friberg in "Food Emulsions", M. Dekker, New York 1976, Mikroemulsionssysteme zur Anwendung bei Nahrungsmitteln. Er vertritt die Auffassung, daß es sich nicht um Emulsionen handelt, d.h. nicht um klassische Zweiphasensysteme mit einer dispersen Phase, die sich aufgrund ihres Grenzflächenpotentials stabilisiert, sondern um mizellare Lösungen, also einphasige Systeme. Auch die von ihm verwendeten hochdispersen Systeme werden jedoch unter Einsatz toxischer Tenside hergestellt.

Die Auffassung, es handele sich bei der Struktur der Systeme um mizellare, einphasige Lösungen, wird in der neueren Literatur immer häufiger aufgegriffen, vgl. J.D. Robb, "Microemulsions", Plenum Press, New York 1982. Es werden dabei aber immer die klassischen Mikroemulsionssysteme mit ionogenen oder nicht-ionogenen Tensiden und Co-Tensiden aus aliphatischen, einwertigen Alkoholen einbezogen.

Eine wäßrige Mikroemulsion mit hautverträglichen Hilfsstoffen zur pharmazeutischen Anwendung wird in der DE-OS 32 12 053 beschrieben. Es werden Emulgatorsysteme aus Estern aliphatischer ($C_3$-$C_{18}$) Alkohole mit aliphatischen ($C_{10}$-$C_{22}$) Carbonsäuren oder aliphatischen ($C_{12}$-$C_{22}$) Alkoholen als Lösungsmittel, Estern mit mindestens einer freien Hydroxylgruppe aus einem Polyethylenglykol-(PEG)-glycerylether mit 2 bis 7 PEG-Einheiten und einer aliphatischen ($C_6$-$C_{22}$) Carbonsäure oder einem Monoether aus einem Polyethylenglykol und einem aliphatischen ($C_{12}$-$C_{18}$) Alkohol als Emulgator sowie aliphatischen ($C_{12}$-$C_{22}$) Alkoholen oder Glycerinestern aliphatischer ($C_6$-$C_{22}$) Carbonsäuren als Co-Emulgator beansprucht.

In der Beschreibung und den Beispielen der DE-OS 32 12 053 werden als bevorzugte Ausführungsform wäßrigen Mikrogele beschrieben, die als Tensid einen Monoether aus PEG und einem aliphatischen ($C_{12}$-$C_{18}$) Alkohol und als Co-Tensid einen aliphatischen ($C_{12}$-$C_{22}$) Alkohol enthalten.

Die Kombination der PEG-Ether und Ester mit aliphatischen Alkoholen zur Senkung der Grenzflächenspannung auf Werte unter 1 mN m$^{-1}$ und damit zur Bildung von sogenannten Mikroemulsionen ist in der Literatur an sich bereits beschrieben, vgl. B.W. Müller, "Receuil des Conferences du 17e Colloque de Pharmacie Industrielle", Genf 1978, Seiten 34 - 36. Ferner sind die beanspruchten Ölkomponenten wie Hexyllaurat, Isopropylmyristat und -laurat aus der Salben- und Makroemulsionstechnologie als Gleitschienen bekannt.

Auch die in der DE-OS 32 12 053 angegebenen Verfahren zur Herstellung der Mikrogele unterscheiden sich bis auf die nicht erforderliche Homogenisierung nicht von der Herstellung von Makroemulsionssystemen mit Mischemulgatoren. Mit den in den Beispielen angegebenen Mischungen werden transparente halbfeste Gelsysteme erhalten, deren Bezeichnung als Mikroemulsion irreführend ist.

Es ist ferner bekannt, daß die Verwendung von gesättigten Alkoholen mit einer Kettenlänge von mehr als 10 Kohlenstoffatomen als Co-Tensid wegen der geringen Diffusionsgeschwindigkeit dieser Alkohole in die Grenzfläche die Bildung von Mikroemulsionen bei Zimmertemperatur nicht in einem ausreichend breiten Konzentrationsbereich zuläßt, vgl. K.S. Birdi, Colloid & Polymer Sci. 260, 628 (1982); S. Candau u. R. Zana, Colloid Sci. 84, 206 (1981). Wäre dem nicht so, so hätten alle Probleme durch die Verwendung von Laurylalkohol, dem ersten nicht-toxischen Alkohol der Reihe, als Co-Tensid gelöst werden können. Die Bildung einer Mikroemulsion bei Zimmertemperatur ist jedoch erst mit Dodekanol möglich.

Es ist Aufgabe der Erfindung, pharmazeutische Mehrkomponentensysteme zu schaffen, die bei Zimmertemperatur stabil und physiologisch unbedenklich sind und die die biologische Verfügbarkeit der in ihnen enthaltenden Wirkstoffe gegenüber bekannten Systemen verbessern.

Zur Lösung der Aufgabe werden die flüssigen transparenten Mehrkomponentensysteme vorgeschlagen, die einen oder mehrere Wirkstoffe in einem System aus einer Ölkomponente, Tensiden, Co-Tensiden und gegebenenfalls Wasser enthalten und dadurch gekennzeichnet sind, daß sie bis zu 70 Gew.% der Ölkomponente, 2 bis 60 Gew.% Tenside, bis zu 78 Gew.% Co-Tenside und gegebenfalls bis zu 85 Gew.% Wasser enthalten, wobei die Summe aus Ölkomponenten und Co-Tensiden mindestens 10 Gew.% beträgt und sie als Tenside physiologisch verträgliche, nicht-ionische Tenside mit einem HLB-Wert größer als 8 oder amphotere Tenside und als Co-Tenside unter 40°C flüssige Partialester und/oder -ether mehrwertiger Alkohole - ausgenommen solcher mit weniger als drei Hydroxygruppen - mit gesättigten oder ungesättigten Fettsäuren und/oder Fettalkoholen mit 6 bis 22 C-Atomen oder Gemische derselben enthalten, wobei gegebenenfalls die Co-Tenside gleichzeitig als Ölkomponente in dem System dienen können, oder die Ölkomponente gegebenenfalls gleichzeitig als Co-Tensid dienen kann, wenn als Ölkomponente Oleylalkohol und/oder polare Ester von einwertigen Alkoholen mit 2 bis 4 C-Atomen mit Fettsäuren mit 8 bis 18 C-Atomen enthalten sind, wobei Isopropyllaurat und Dodecanol gleichzeitig enthaltende Systeme ausgenommen sind, und die einzelnen Komponenten in dem System in solchen Mengen vorhanden sind, daß bei Zimmertemperatur stabile, flüssige, einphasige Systeme gebildet werden, die den oder die Wirkstoffe gelöst enthalten.

Es hat sich gezeigt, daß die Penetration eines pharmazeutischen Wirkstoffes durch die Haut beschleunigt wird, wenn man diesen gelöst appliziert. Dabei bleibt der Depotcharakter der Hornhautschicht erhalten und die Passage in und durch die Membran wird gefördert, so daß auch Moleküle mit kleinen Diffusionskoeffizienten aufgenommen werden. Werden die erfindungsgemäßen Systeme peroral oder parenteral verabreicht, so tritt bei der Verdünnung mit der wäßrigen Phase, nämlich Magensaft bzw. Serum, eine Übersättigung der Lösung mit Wirkstoff ein, und es kann auf diese Weise eine gute Verfügbarkeit des Arzneistoffes erzielt werden.

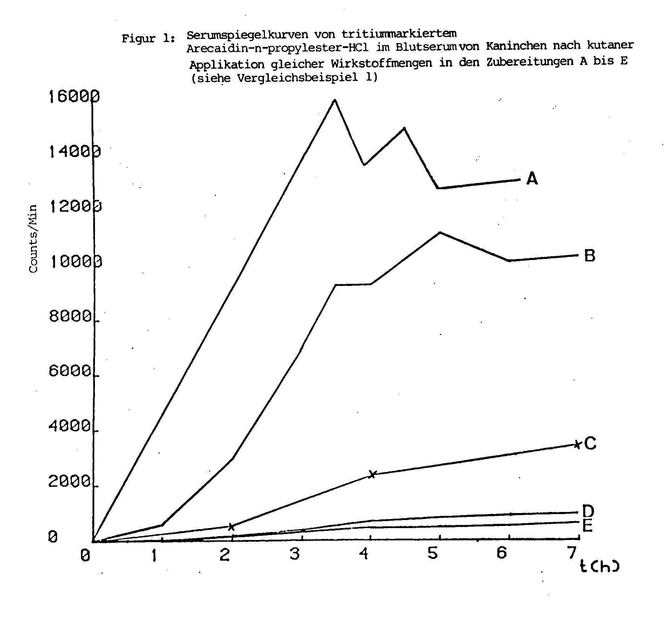

Gegenüber den in der DE-OS 32 12 053 beschriebenen Gelsystemen konnte erfindungsgemäß eine überraschende Steigerung der biologischen Verfügbarkeit sowohl aus den wasserfreien, ölreichen als auch den wasserreichen, dünnflüssigen Systemen erzielt werden (siehe Vergleichsbeispiel 1 und Figur 1). Dies wird vermutlich zum einen durch die geringe Viskosität und die grenzflächenaktiven Eigenschaften des Systems bewirkt, die zu einer schnellen Penetration durch die Hornschicht führen, und zum anderen dadurch, daß durch Überschreiten der Sättigungskonzentration des Wirkstoffes in dem System durch Änderung der Wasserkonzentration eine erhöhte thermodynamische Aktivität des Wirkstoffes eintritt.

Wasserfreie Lösungen, welche liphophile Wirkstoffe gelöst enthalten, werden vorzugsweise unter Okklusivbedingungen, z.B. mittels eines undurchlässigen Pflasters, auf die Haut aufgebracht. Das aus der Haut in das System eindringende Wasser vermindert die Löslichkeit des Wirkstoffes und führt dadurch zu einer übersättigten Lösung. Wenn man andererseits wasserlösliche Wirkstoffe in einem wasserhaltigen System löst und dieses auf die Haut appliziert, kommt es durch Verdampfen eines Teils des Wassers ebenfalls zu einer Übersättigung an Wirkstoff.

Die besonderen Eigenschaften der erfindungsgemäßen Systeme beruhen auf der Verwendung bestimmter Tensid/Co-Tensidkombinationen. Als Tensid wird erfindungsgemäß ein polyoxyethylierter Glycerinester und/oder -ether mit aliphatischen ($C_6$-$C_{22}$)-Carbonsäuren bzw. -Alkoholen und mit mehr als 7 bis maximal 40 Ethylenoxideinheiten verwendet. Ferner sind grundsätzlich alle nicht-ionogenen Tenside mit einem HLB-Wert oberhalb 8, insbesondere PEG-Glycerinester, PEG-Fettsäureester, PEG-Fettalkoholether, ethoxylierte Cyclopentanoperhydrophenanthren-Derivate mit 5 bis 50 Ethylenoxideinheiten, ethoxylierte Cholesterole, Zuckerester und Ethylenoxid-Propylenoxid-Blockpolymere als Tensidkomponente geeignet. Ferner eignen sich als Tenside amphotere Verbindungen wie Fettsäure-amido-alkyl-betaine mit $C_2$-$C_{22}$-Fettsäuren oder Lecithinderivate.

Die Bildung der erfindungsgemäßen Systeme ist jedoch nur in Kombination mit bestimmten Co-Tensiden, nämlich Partialestern oder -ethern mehrwertiger Alkohole - ausgenommen solcher mit weniger als drei Hydroxy grüppen - mit Fettsäuren oder Fettalkoholen mit 4 bis 22 Kohlenstoffatomen, insbesondere mit 8 bis 10 Kohlenstoffatomen, sowie deren Gemischen möglich. Auf die Co-Tenside kann nur dann verzichtet werden, wenn als Ölkomponente Oleylalkohol und/oder polare Ester von einwertigen Alkoholen mit 2 bis 4 C-Atomen mit Fettsäuren mit 8 bis 18 C-Atomen und als Tensidkomponente niedermolekulare, Polyoxyethylen/Polyoxypropylencopolymerisate, bevorzugt mit einem PEG-Anteil von 20-50 Gew. %, bezogen auf das Copolymere, und einem Molekulargewicht des Polypropylenanteils von 600 bis 1400, vorhanden sind.

Nachfolgend sind Beispiele für erfindungsgemäß bevorzugte Mehrkomponentensysteme angegeben:

1. Zubereitungen aus PEG(20 EO)-Ölsäureglycerinpartialestern, Capryl-Caprinsäureglycerinpartialestern oder mittelkettigen Triglyceriden, Isopropylpalmitat und Wasser.

2. Zubereitungen aus PEG-Sojasterin (25 EO), Capryl-Caprinsäurepartialestern, Isopropylmyristat und Wasser.

3. Zubereitungen aus PEG(20 EO)-Laurinsäureglycerinpartialestern, Capryl-Caprinsäureglycerinpartialestern, Isopropylpalmitat und Wasser.

4. Zubereitungen aus Polyoxyethylen/Polyoxypropylen-Copolymerisaten mit einem Molekulargewicht des Polyoxypropylenanteils von 1200 und einem Polyoxyethylenanteil von 20 bis 40 Gew.% bezogen auf das Gesamtgewicht des Copolymeren, Oleylalkohol und Wasser. In diesem System bildet der Oleylalkohol sowohl die Ölkomponente als auch das Co-Tensid.

Als "Ölkomponente" sind erfindungsgemäß lipophile, mit Wasser nicht mischbare Flüssigkeiten anzusehen, während unter den Begriff "wäßrige Komponente" hydrophile, polare Flüssigkeiten fallen.

Als Ölkomponenten eignen sich dünn- und dickflüssige aliphatische Kohlenwasserstoffe, synthetische und natürliche Öle wie Olivenöl, Erdnußöl, Rapsöl, etc. sowie insbesondere Ölsäureoleylester, Isooctylstearat, Laurinsäurehexylester, Di-n-butyladipat, Isopropylmyristat, -palmitat, -stearat, Triglyceridgemische aus gesättigten und ungesättigten Fettsäuren mit 4 bis 22 Kohlenstoffatomen, Oleylalkohol, etherische Öle, Isopropylcaprylat, Isopropylcaprinat und Isopropyllaurat.

Abhängig von dem eingesetzten Mischungsverhältnis und der Art der verwendeten Tensidkomponenten lassen sich Einphasenbereiche mit unterschiedlichen Wasserkonzentrationen herstellen.

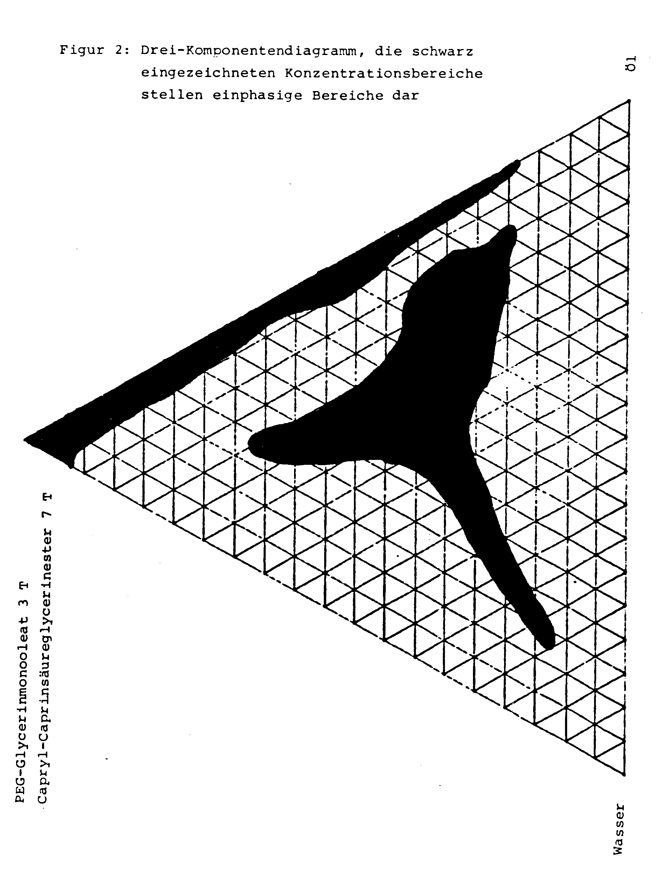

Figur 2 zeigt ein Dreikomponenten-Diagramm für das System Wasser/Isopropylstearat mit einem 3 : 7-Gemisch aus PEG(20)-Glycerinmonooleat und Caprin-Caprylsäureglycerinester mit 40 % Monoglyceridanteil. In dem Diagramm stellen die gefärbten Bereiche die Einphasenbereiche bei Raumtemperatur (28°C) dar.

Art und Konzentration des Mischemulgators aus Tensid und Co-Tensid bestimmen sowohl den Umfang des thermodynamisch stabilen Einphasenbereiches als auch die Viskosität des Systems und den Temperaturbereich, in dem die thermodynamische Stabilität gegeben ist. Zur pharmazeutischen Anwendung ist ein System mit möglichst geringer Tensidkonzentration in einem Temperaturbereich bis mindestens 40°C am besten geeignet. Wird ein einphasiges System ohne Änderung der Konzentration der Komponenten erwärmt, so schlägt es je nach Zusammensetzung bei einer bestimmten Temperatur in ein mehrphasiges System um, wobei sich die einzelnen Phasen in verschiedener Weise abscheiden. Bei Abkühlung bildet sich das ursprüngliche, einphasige System zurück. Ebenso verhalten sich die Systeme zum tieferen Temperaturbereich hin. Auch hier bildet sich nach Wiederauflösen der auskristallisierten Tenside das ursprüngliche transparente System zurück.

Die erfindungsgemäßen, flüssigen, transparenten Mehrkomponentensysteme können hergestellt werden, indem man zunächst Tensid, Co-Tensid und Ölphase miteinander vermischt und den oder die Wirkstoffe in dieser Mischung löst. Zum Lösen der festen Tenside sowie des/der pharmazeutischen Wirkstoffe(s) kann leichtes Erwärmen von Nutzen sein. Die wäßrige Phase wird, falls vorhanden, zum Schluß bei Zimmertemperatur eingearbeitet. Bei der Herstellung der erfindungsgemäßen Systeme ist es gegenüber den bekannten Gelen und Makroemulsionen vorteilhaft, daß die Herstellung ohne wesentlichen Energieaufwand erfolgt und eine Homogenisierung nicht notwendig ist.

Durch Auswahl und Konzentration der verwendeten Komponenten sind Viskosität und Temperaturbereich, bei denen die Zubereitung stabil ist, steuerbar.

Da der gelöste Wirkstoff in dem erfindungsgemäßen System als zusätzliche Komponente wirkt, wird je nach dem Charakter der Substanz der Konzentrationsbereich des Einphasengebietes verschoben. Gesetzmäßigkeiten konnten bisher nicht abgeleitet werden; es muß daher für jeden Wirkstoff die optimale Konzentration der Komponenten Wasser, Öl und Mischemulgator neu bestimmt werden. Zu diesem Zweck wird eine Mischung aus 30 bis 50 Gew.% Öl mit dem Tensid und dem CoTensid im Verhältnis von etwa 2 : 3 zusammengegeben, worauf in diesem System der jeweilige Wirkstoff bis zur Sättigung gelöst wird. Anschließend fügt man etwa 50 Gew.% Wasser zu und beobachtet, ob eine Wasserabscheidung oder eine Ölabscheidung stattfindet. Im ersteren Fall wird weiteres Co-Tensid, im zweiten Fall weiteres Tensid zugesetzt, bis sich ein einphasiges System bildet. Auf diese Weise ist es leicht möglich, für jedes System die optimale Zusammensetzung zu ermitteln.

In der Regel enthalten die erfindungsgemäßen Systeme

bis 70 Gew.% einer Ölkomponente

bis 85 Gew.%, z.B 35-85 Gew. % einer wäßrigen Komponente

5 bis 60 Gew.% eines Mischemulgators aus einem Tensid mit einem HLB>8 (5 bis 95 Gew.% bezogen auf den Mischemulgator) und einem Co-Tensid mit einem HLB<8 (5 bis 95 Gew.% bezogen auf den Mischemulgator).

Die jeweilige Wirkstoffkonzentration richtet sich nach den therapeutischen Erfordernissen sowie der Sättigungslöslichkeit des Wirkstoffs in dem System. Es ist bereits von den kolloidchemisch gut untersuchten ionogenen Mischemulgatorsystemen bekannt, daß die Ölkomponente sich in der Grenzschicht an der Co-Tensidfunktion beteiligen kann, vgl. S. Levine u. K. Robinson, J. Phys. Chem. 76, 877 (1972); J.R. Hansen, J. Phys. Chem. 73, 256 (1974). In ähnlicher Weise wirken die beschriebenen Ölkomponenten in dem erfindungsgemäßen System, so daß sich der Einphasenbereich je nach der Zusammensetzung der Ölphase verschiebt und unter Umständen der Co-Tensidanteil bzw. der Ölanteil reduziert und auf letzteren sogar verzichtet werden kann.

Als Ölkomponenten sind physiologisch verträgliche, bei Zimmertemperatur flüssige, lipophile Verbindungen geeignet, insbesondere

a) natürliche Öle wie Erdnußöl, Olivenöl, Rizinusöl, Sesamöl, Rapsöl, etherische Öle,

b) halbsynthetische und synthetische Mono-, Di- und Triglyceride aus gesättigten und ungesättigten ($C_6$-$C_{22}$) Fettsäuren sowie ihre ethoxylierten Derivaten;

c) flüssige Wachse wie Isopropylmyristat, -caprinat, -caprylat, -laurat, -palmitat und -stearat; Ölsäureester z.B. Olsäureoleylester, Ethyloleat;

d) ein- und mehrwertige aliphatische Alkohole wie Hexadecylalkohol, 2-Octyldodecanol, Oleylalkohol etc. sowie ihre ethoxylierten Derivate, wobei Isopropyl Laurat und Dodecanol gleichzeitig enthaltende Systeme erfindungsgemäß ausgenommen sind ;

e) aliphatische ($C_4$-$C_{10}$) Carbonsäuren sowie ihre Ethoxyderivate.

Die erfindungsgemäßen Systeme können ferner Stabilisatoren enthalten. Diese können neben Antioxidantien und Konservierungsmitteln auch Puffersubstanzen und Isotonisierungsmittel sein. Darüber hinaus können auch zur chemischen Stabilisierung des Wirkstoffs oder des Wirkstoffsgemisches spezielle Stabilisatoren wie z.B. Weinsäure in Verbindung mit Ergotamintartrat oder Natriumpolyphosphat in Verbindung mit Phenylbutazon enthalten sein.

In die erfindungsgemäßen Systeme können alle geeigneten Wirkstoffe sowohl für lokale als auch für systemische Wirkung eingearbeitet werden. Die Anwendung ist kutan, peroral, parenteral, vaginal oder rektal möglich. Bei kutaner Applikation erfolgt die Wirkungssteuerung sowohl lokal als auch systemisch über die Konzentration der Wasserphase, die Natur der Ölkomponente und die Zusammensetzung des Mischemulgators. In einer Zubereitung des erfindungsgemäßen Systems zur transdermalen Anwendung hydrophiler Wirkstoffe oder zur kutanen, lokalen Anwendung lipophiler Wirkstoffe liegen die Komponenten in folgenden Konzentrationen vor:

0,01    bis 15 Gew.% fester Wirkstoff bzw. bis zu 65 Gew.% flüssiger Wirkstoff

2,5    bis 40 Gew.% Tensid

0    bis 45 Gew.% Co-Tensid

0    bis 35 Gew.% Ölkomponente

40    bis 80 Gew.% Wasser,
        wobei die Summe der Co-Tensid- und Ölkomponenten mindestens 2,5 Gew.% beträgt.

Statt Wasser können auch aliphatische ein- oder mehrwertige $C_2$-$C_4$-Alkohole oder Harnstoff allein, in Kombination oder in Kombination mit Wasser Verwendung finden.

Ziel ist dabei für lipophile Wirkstoffe, die nicht systemisch wirken sollen, die Zubereitung eines Systems, das schnell in das Stratum corneum penetriert und zu einer Sättigung an Wirkstoff führt. Beispiele für auf diese Weise verwendbare Wirkstoffe sind Cortisone, Antipsoriatika, Antimykotika, Salicylate, Zytostatika, Antibiotika, Virustatika, Antihistaminika, UV-Absorber, Chemotherapeutika, Antiseptika, Oestrogene, Antihidrotika, etherische Öle und Narbenbehandlungsmittel.

In erfindungsgemäßen Systemen zur transdermalen (systemischen) Anwendung lipophiler Wirkstoffe können die Komponenten in den folgenden Konzentrationen vorliegen:

0,1 bis 15 Gew.% fester Wirkstoff bzw. bis bis 65 Gew.% flüssigen Wirkstoff

1 bis 50 Gew.% Tensid

0 bis 80 Gew.% Co-Tensid

0 bis 85 Gew.% Ölkomponente

wobei die Summe der Co-Tensid- und Ölkomponenten mindestens 2,5 Gew.% beträgt.

Beispiele für erfindungsgemäß systemisch anwendbare Wirkstoffe sind Antirheumatika, Antiphlogistika, β-Blocker, Antiemetika, Antiarrythmika, Anthelmintika, Kardiaka, Spasmolytika, Thrombozytenaggregationshemmer, etherische Öle und alle Substanzen mit einem hohen First-Pass-Effekt.

Die Auftragung der erfindungsgemäßen Systeme auf die Haut kann z.B. per Dosieraerosol oder Dosierspray sowie als Pflaster erfolgen. In letzterem Fall wird die flüssige Zubereitung von den Poren eines schwammartigen Trägervlieses in dem Reservoir einer Pflasterzubereitung aufgenommen. Dieser Träger wird nach dem Abziehen der Schutzfolie direkt ohne Steuermembran auf die Haut gebracht. Die kolloidale Lösung migriert in Abhängigkeit von ihrer Zusammensetzung mit einer bestimmten Geschwindigkeit in das Stratum corneum und dieses sorgt für einen konstanten Wirkstofftransport. Das Stratum corneum übernimmt quasi die Funktion einer Steuermembran, wobei jedoch die Steuerung durch die Zusammensetzung der Zubereitung bedingt ist. Die erfindungsgemäßen Systeme zur oralen und parenteralen Anwendung unterscheiden sich hinsichtlich der Konzentration der Komponenten wenig von den vorstehend beschriebenen Systemen. Ziel ist hier die Zubereitung von Systemen, die bei einer Verdünnung mit Magensaft oder Serum sehr feindisperse O/W-Makroemulsionen bilden, die bis zur Sättigungslöslichkeit den Wirkstoff einschließen und retardiert freisetzen. Man kann diese hochdispersen Systeme aufgrund ihres Grenzflächenverhaltens auch selbstemulgierend nennen, und sie bilden feindisperse Emulsionen mit einer annähernd konstanten Teilchengrößenverteilung der inneren Phase.

Für die perorale Anwendung beispielsweise als Tropfen oder in Gelatinekapseln ist eine organoleptische Auswahl der Tenside und Co-Tenside erforderlich, während bei parenteraler Anwendung darauf zu achten ist, daß die Tenside keine wesentliche hämolytische Aktivität entfalten.

In einer weiteren Ausführungsform der Erfindung können auch solche Systeme verwendet werden, die keine Ölphase mehr besitzen und bei denen nur die Co-Tensidkomponente lipophiler Natur ist. Diese sind zur oralen, parenteralen, perkutanen und insbesondere zur rektalen Anwendung geeignet.

In derartigen Systemen sind die Grenzen für die Konzentrationsbereiche der Komponenten erheblich enger:

1 bis 5 Gew.% Wirkstoff

3 bis 30 Gew.% Tensid

10 bis 45 Gew.% Co-Tensid

50 bis 70 Gew.% Wasser.

Die Verabreichung der Zubereitungen kann entweder als Klistier oder bei Rezepturen mit niedrigem Wasseranteil auch als Rektalkapsel erfolgen.

Die Erfindung wird nachfolgend anhand von Beispielen erläutert.

Beispiel 1

Es wurden verschiedene erfindungsgemäße Mehrkomponentensysteme zubereitet, indem wenn nicht anders angegeben zunächst eine Mischung aus Tensid, Co-Tensid, Ölkomponente und gegebenenfalls Wirkstoff bei Zimmertemperatur hergestellt und anschließend gegebenenfalls das Wasser zugegeben wurde.

Es wurden folgende Zubereitungen hergestellt:

Zubereitung I:

| | |
|---|---|
| PEG (20 EO)-Ölsäureglycerinpartialester | 40 Gew.% |
| Capryl-Caprinsäureglycerinpartialester (42 % Monoglyceridgehalt) | 24 Gew.% |
| mittelkettige Triglyceride | 16 Gew.% |
| Wasser | 20 Gew.% |

Zubereitung II:

| | |
|---|---|
| PEG (20 EO)-Ölsäureglycerinpartialester | 25 Gew.% |
| Capryl-Caprinsäureglycerinpartialester (42 % Monoglyceridgehalt) | 15 Gew.% |
| mittelkettige Triglyceride | 10 Gew.% |
| Wasser | 50 Gew.% |

Zubereitung III:

| | |
|---|---|
| Isosorbiddinitrat | 3,5 Gew.% |
| PEG (20 EO)-Ölsäureglycerinpartialester | 23,2 Gew.% |
| Capryl-Caprinsäureglycerinpartialester (42 % Monoglyceridgehalt) | 34,8 Gew.% |
| Isopropylpalmitat | 38,5 Gew.% |

Das Isosorbiddinitrat wurde unter Erwärmen in der Mischung aus Tensid, Co-Tensid und Ölkomponente gelöst.

Zubereitung IV:

| | |
|---|---|
| PEG (20 EO)-Ölsäureglycerinpartialester | 24 Gew.% |
| Capryl-Caprinsäureglycerinpartialester (40 % Monoglyceridgehalt) | 16 Gew.% |
| 1,2-Propylenglykol | 60 Gew.% |
| Wasser | beliebig |

Zubereitung V:

| | |
|---|---|
| Indometacin | 2,5 Gew.% |
| PEG (20 EO)-Ölsäureglycerinpartialester | 24 Gew.% |
| Capryl-Caprinsäureglycerinpartialester (40 % Monoglyceridgehalt) | 15 Gew.% |
| mittelkettige Triglyceride | 10 Gew.% |
| Wasser | 48,5 Gew.% |

Zubereitung VI:

| | |
|---|---|
| Indometacin | 4,0 Gew.% |
| Polyoxyethylen/Polyoxypropylen-Copolymer (30 Gew.%) (MG 1200) | 48,0 Gew.% |
| Oleylalkohol | 48,0 Gew.% |

Zubereitung VII:

| PEG (20 EO)-Ölsäureglycerinpartialester | 12,5 Gew.% |
| Capryl-Caprinsäureglycerinpartialester (42 % Monoglyceridgehalt) | 23,5 Gew.% |
| Isopropylmyristat (Ölkomponente) | 10,0 Gew.% |
| Eukalyptusöl (Ölkomponente) | 10,0 Gew.% |
| Kiefernadelöl (Ölkomponente) | 10,0 Gew.% |
| Campher | 3,0 Gew.% |
| Menthol | 3,0 Gew.% |
| Wasser | 28,0 Gew.% |

Menthol und Campher wurden bei Zimmertemperatur in der Mischung aus etherischem Öl, Tensid und Co-Tensid gelöst und zum Schluß wurde das Wasser zugegeben.

Diese Zubereitung kann als Balneotherapeutikum oder als Inhalationsmittel eingesetzt werden. In einem handelsüblichen Inhalator mit heißem Wasser versetzt wird die Abgabe des etherischen Öls in die Dampfphase durch den mizellaren Einschluß bei der Bildung der Makroemulsion gegenüber dem Einsatz einer gleichen etherischen Ölzusammensetzung in einer Salbengrundlage bis um das 5-Fache verlängert.

Zubereitung VIII:

| Polypeptid Hirudin (10 000 ATE = 0,7 g) | 0,073 Gew.% |
| PEG (25 EO)-Sojasterin | 26,0 Gew.% |
| Capryl-Caprinsäureglycerinpartialester (40 % Monoglyceridgehalt) | 30,8 Gew.% |
| Isopropylpalmitat | 30,9 Gew.% |
| Wasser | 12,227 Gew.% |

Zubereitung IX:

| PEG (20 EO)-Ölsäureglycerinpartialester | 11,0 Gew.% |
| Caprylsäuremonoglycerid (90 % Monoglyceridgehalt) | 16,0 Gew.% |
| Isopropylstearat | 18,0 Gew.% |
| Wasser | 55,0 Gew.% |

Zubereitung X:

| Isopropanol | 20 Gew.% |
| Lecithin | 10 Gew.% |
| Isopropylpalmitat | 40 Gew.% |
| Wasser | 30 Gew.% |

Zubereitung XI:

| Sojaphosphatide (70 bis 75 % Phosphatidylcholin) | 6,9 Gew.% |
| Isopropylpalmitat | 24,9 Gew.% |
| Isopropanol | 28,0 Gew.% |
| Wasser | 42,0 Gew.% |

Beispiel 2

In vitro Nachweis der Absorption eines Makromoleküls durch aktiven Transport aus dem erfindungsgemäßen hochdispersen Mehrkomponentensystem.

5 ml der Zubereitung VIII gemäß Beispiel 1 wurden in einer Sartoriuszelle mit einer Oberfläche von 87,5 cm² 10 ml eines Phosphatpuffers mit einem pH-Wert von 7,6 als künstlicher Blutphase gegenübergestellt. Als Membran wurde frische, ungebrühte Schweinehaut etwa 1 Stunde nach dem Tod des Tieres verwendet. Der Versuch wurde bei 32°C durchgeführt und die Konzentration des Hirudins in dem Phosphatpuffer über einen Zeitraum von 48 Stunden gemessen.

Die Ergebnisse sind in Tabelle 1 wiedergegeben.

Tabelle 1

| Zeit (h) | Konzentration (ATE/ml) |
|---|---|
| 2 | 3 - 4 |
| 6 | 12 |
| 12 | 19 |
| 24 | 24 |

Es wurde festgestellt, daß die Hirudinpassage weitgehend unabhängig von der Dicke der verwendeten Schweinehaut verlief. 48 Stunden nach dem Tod des Tieres konnte kein Transport des Makromoleküls mehr beobachtet werden. Die Ergebnisse lassen auf einen aktiven oder trägervermittelten Transport des Polypeptidmoleküls durch die noch lebende Schweinehaut schließen.

Beispiel 3

Indometacin-Aufnahme nach oraler Verabreichung des Wirkstoffes in dem erfindungsgemäßen hochdispersen Mehrkomponentensystem beim Menschen.

Bei männlichen Probanden wurde jeweils 1 g der Zubereitung V gemäß Beispiel 1 in einer Hartgelatinekapsel oral verabreicht. Der Indometacinspiegel im Serum wurde anschliessend mittels Hochdruckflüssigkeits-Chromatographie bestimmt.

Die Ergebnisse sind in Tabelle 2 wiedergegeben.

Tabelle 2

| Zeit nach Verabreichung | Plasmakonzentration ($\mu$g ml$^{-1}$) |
|---|---|
| 30 min | 0,6 ± 0,3 |
| 60 min | 1,0 ± 0,4 |
| 120 min | 1,7 ± 0,6 |
| 180 min | 2,5 ± 0,6 |
| 4 h | 2,2 ± 0,7 |
| 6 h | 1,8 ± 0,5 |
| 8 h | 1,5 ± 0,4 |
| 12 h | 0,7 ± 0,2 |

Die Ergebnisse lassen deutlich einen Retardeffekt erkennen.

Vergleichsbeispiel 1

Es wurde die Aufnahme von tritiummarkiertem Arecaidin-n-propylester-HCl aus den folgenden Systemen untersucht:

A Zubereitung des Beispiels 1 (ölreich)

B Zubereitung II des Beispiels 1 (wasserreich)

C Mikrogel gemäß DE-OS 32 12 053 der folgenden Zusammensetzung

9

| PEG-oleylether (Brij 97) | 23,2 Gew.% |
| Isopropyllaurat | 23,2 Gew.% |
| Dodecanol | 7,3 Gew.% |
| Wasser | 46,3 Gew.% |

D Makroemulsion unter Verwendung der gleichen Komponenten wie in den Zubereitungen I und II des Beispiels 1:

| PEG (20 EO)-Ölsäureglycerinpartialester | 25 Gew.% |
| Capryl-Caprinsäureglycerinpartialester | 15 Gew.% |
| mittelkettige Triglyceride | 15 Gew.% |
| Wasser | 45 Gew.% |

E wasserhaltige Wollwachsalkoholsalbe gemäß DAB 8.

In jede der Zubereitungen wurde tritiummarkiertes Arecaidin-n-propylester-HCl in einer solchen Menge eingearbeitet, daß eine Radioaktivität von 1 µCi/4g Zubereitung erhalten wurde. Jeweils 3,5 g jeder Zubereitung wurden bei drei Kaninchen (Gewicht 3,5 kg) auf 100 cm$^2$ rasierte Haut aufgetragen. Anschließend wurde die Tritiumaktivität im Serum der behandelten Tiere an jeweils 0,2 ml Serum im Abstand von 1 Stunde insgesamt 7 Stunden lang gemessen. Für jede Zubereitung wurden die Mittelwerte der an drei Tieren gewonnenen Meßergebnisse in counts/min ermittelt. Die Ergebnisse sind in Figur 1 in graphischer Darstellung wiedergegeben.

Es zeigt sich, daß die biologische Verfügbarkeit des applizierten Wirkstoffes aus dem erfindungsgemäßen System signifikant höher ist, als aus den vorbekannten Systemen. Dies gilt sowohl für die ölreiche (A) als auch für die wasserreiche (B) erfindungsgemäße Zubereitung.

Der Verlauf der Serumspiegelkonzentrationen deutet einen Steady State und damit eine retardierte Freisetzung auf einem gesteigerten Niveau an.

Vergleichsbeispiel 2

Absorption von Isosorbiddinitrat bei kutaner Applikation beim Menschen.

Es wurde die Isosorbiddinitrat enthaltende, erfindungsgemäße Zubereitung III des Beispiels 1 sowie eine den Wirkstoff in gleicher Menge enthaltende handelsübliche Salbenzubereitung (ISOKET$^R$-Salbe) verwendet. Die Zubereitungen wurden jeweils 3 Probanden kutan appliziert. Das erfindungsgemäße hochdisperse System wurde in einer 100 mg Wirkstoff enthaltenden Menge auf 150 cm$^2$ Hautfläche aufgetragen, während die gleiche Wirkstoffmenge in der handelsüblichen Salbenzubereitung auf 400 cm$^2$ aufgetragen wurde.

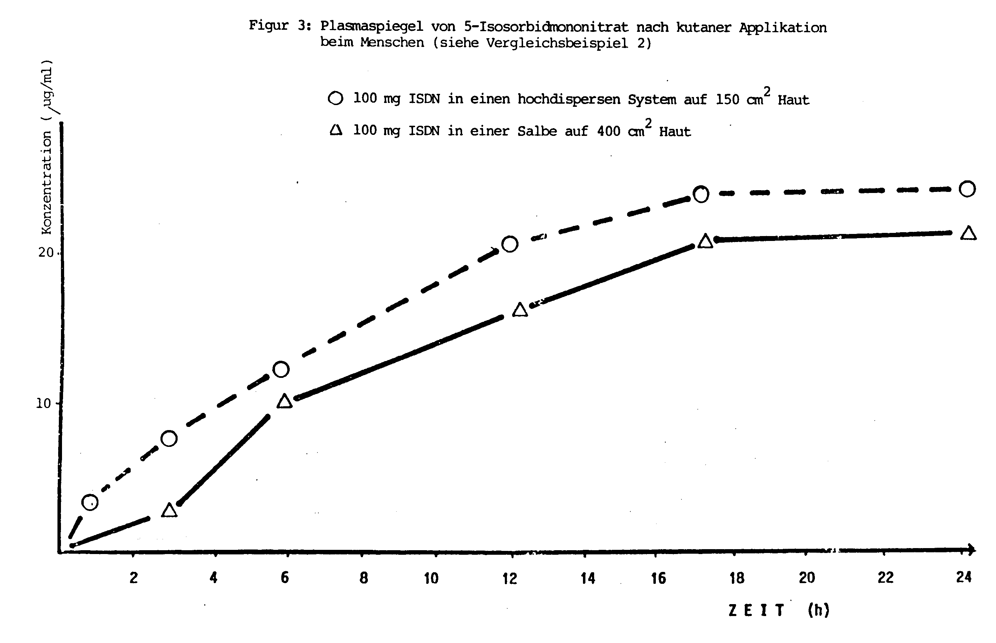

Anschließend wurde die 5-Isosorbidmononitrat-Konzentration im Serum der Probanden gaschromatographisch jeweils 24 Stunden lang bestimmt und die Mittelwerte wurden für jede der Zubereitungen errechnet. Die Ergebnisse sind in Figur 3 in graphischer Darstellung wiedergegeben.

Es zeigt sich, daß die Konzentration des Wirkstoffes im Blutplasma der Probanden nach Auftragung der erfindungsgemäßen Zubereitung deutlich höher liegt als nach Auftragung der handelsüblichen Salbe, obgleich die zur Verfügung stehende Absorptionsfläche bei ersterer um den Faktor 2,6 geringer war.

**Patentansprüche**

**1.** Transparente Mehrkomponentensysteme zur pharmazeutischen Anwendung, die einen oder mehrere Wirkstoffe in einem System aus einer Ölkomponente, Tensiden, Co-Tensiden und gegebenenfalls Wasser enthalten, **dadurch gekennzeichnet**, daß sie bis zu 70 Gew.% der Ölkomponente, 2 bis 60 Gew.% Tenside, bis zu 78 Gew.% Co-Tenside und gegebenfalls bis zu 85 Gew.% Wasser enthalten, wobei die Summe aus Ölkomponenten und Co-Tensiden mindestens 10 Gew.% beträgt und daß sie als Tenside physiologisch verträgliche, nicht-ionische Tenside mit einem HLB-Wert größer als 8 oder amphotere Tenside und als Co-Tenside unter 40°C flüssige Partialester und/oder -ether mehrwertiger Alkohole - ausgenommen solcher mit weniger als drei Hydroxygruppen - mit gesättigten oder ungesättigten Fettsäuren und/oder Fettalkoholen mit 6 bis 22 C-Atomen oder Gemische derselben enthalten, wobei gegebenenfalls die Co-Tenside gleichzeitig als Ölkomponente in dem System dienen können, oder die Ölkomponente gegebenenfalls gleichzeitig als Co-Tensid dienen kann, wenn als Ölkomponente Oleylalkohol und/oder polare Ester von einwertigen Alkoholen mit 2 bis 4 C-Atomen mit Fettsäuren mit 8 bis 18 C-Atomen enthalten sind, wobei Isopropyllaurat und Dodecanol gleichzeitig enthaltende Systeme ausgenommen sind, und die einzelnen Komponenten in dem System in solchen Mengen vorhanden sind, daß bei Zimmertemperatur stabile, flüssige, einphasige Systeme gebildet werden, die den oder die Wirkstoffe gelöst enthalten.

2. Mehrkomponentensysteme nach Anspruch 1, **dadurch gekennzeichnet**, daß sie als Tenside ethoxylierte Glycerinpartialester und/oder -ether mit aliphatischen Carbonsäuren bzw. Alkoholen mit 2 bis 22 C-Atomen und mit mehr als 7 bis maximal 40 Ethylenoxideinheiten enthalten.

3. Mehrkomponentensysteme nach Anspruch 1, dadurch gekennzeichnet, daß sie als Tenside Polyethylenglykolester und/oder -ether mit aliphatischen Carbonsäuren mit 6 bis 22 C-Atomen bzw. mit aliphatischen Alkoholen mit 6 bis 22 C-Atomen enthalten.

4. Mehrkomponentensysteme nach Anspruch 1, dadurch gekennzeichnet, daß sie als Tenside ethoxylierte Cyclopentanoperhydrophenanthren-Derivate mit 5 bis 50 Ethylenoxideinheiten enthalten.

5. Mehrkomponentensysteme nach Anspruch 1, dadurch gekennzeichnet, daß sie als Tenside Polyoxyethylen/Polyoxypropylen-Copolymerisate enthalten.

6. Mehrkomponentensysteme nach Anspruch 1, dadurch gekennzeichnet, daß sie als Tenside Fettsäureamidoalkyl-betaine mit ($C_2$-$C_{22}$) Fettsäuren oder Lecithin enthalten.

7. Mehrkomponentensysteme nach den Ansprüchen 1 bis 6, dadurch gekennzeichnet, daß sie als Co-Tenside Partialester und/oder -ether mehrwertiger Alkohole mit gesättigten Fettsäuren und/oder Alkoholen mit 8 bis 10 C-Atomen sowie deren Gemische enthalten.

8. Mehrkomponentensysteme nach den Ansprüchen 1 bis 7, dadurch gekennzeichnet, daß sie als Co-Tenside Glycerinpartialester und/oder -ether mit Fettsäuren und/oder Fettalkoholen mit 8 bis 10 C-Atomen enthalten.

9. Mehrkomponentensysteme nach den Ansprüchen 1 bis 8, dadurch gekennzeichnet, daß sie als Co-Tenside Caprin- und/oder Caprylsäureglycerinester, gemischte Caprin-Caprylsäureglycerinpartialester sowie Gemische derselben enthalten.

10. Mehrkomponentensysteme nach Anspruch 1, dadurch gekennzeichnet, daß sie als Tenside PEG-(20 EO)-Ölsäureglycerinpartialester und als Co-Tenside Caprin-Caprylsäureglycerinpartialester enthalten.

11. Mehrkomponentensysteme nach den Ansprüchen 1 bis 10, dadurch gekennzeichnet, daß sie als Ölkomponente Oleylalkohol und/oder polare Ester von einwertigen Alkoholen mit 2 bis 4 C-Atomen mit Fettsäuren mit 8 bis 12 C-Atomen und als Tenside niedermolekulare Polyoxyethylenpolyoxypropylencopolymerisate mit einem PEG-Anteil von 20 - 50 Gew.%, bezogen auf das Copolymer, und einem Molekulargewicht des Polypropylenanteils von 600 bis 1400 enthalten, wobei gegebenenfalls die Ölkomponente in dem System gleichzeitig als Co-Tensid dienen kann.

12. Mehrkomponentensysteme nach den Ansprüchen 1 bis 11, dadurch gekennzeichnet, daß sie zusätzlich Wasser und/oder eine oder mehrere hydrophile Komponenten enthalten.

13. Mehrkomponentensysteme nach Anspruch 12, dadurch gekennzeichnet, daß sie als hydrophile Komponente einen aliphatischen ein- oder mehrwertigen $C_2$- bis $C_4$-Alkohol oder Harnstoff allein oder in Kombination oder in Kombination mit Wasser enthalten.

14. Mehrkomponentensysteme nach den Ansprüchen 1 bis 13, **dadurch gekennzeichnet**, daß sie 35 bis 85 Gew.% Wasser enthalten.

15. Verfahren zur Herstellung der Mehrkomponentensysteme nach den Ansprüchen 1 bis 14, **dadurch gekennzeichnet**, daß man die Tenside und Co-Tenside sowie gegebenenfalls die Ölkomponente miteinander vermischt, anschließend den pharmazeutischen Wirkstoff, gegebenenfalls unter Erwärmen, in der Mischung löst und gegebenenfalls zum Schluß bei Zimmertemperatur das Wasser hinzufügt.

**16.** Verwendung der Mehrkomponentensysteme nach den Ansprüchen 1 bis 14 zur Herstellung von pharmazeutischen Zubereitungen zur kutanen, peroralen, parenteralen, vaginalen oder rektalen Verabreichung sowie von Inhalationen, Balneotherapeutika sowie kosmetischen Präparaten.

**17.** Verwendung der Mehrkomponentensysteme zur Herstellung von pharmazeutischen Zubereitungen nach Anspruch 16, **dadurch gekennzeichnet**, daß man zur transdermalen Applikation liphophiler Wirkstoffe ein wasserfreies Okklusivsystem herstellt.

**18.** Verwendung der Mehrkomponentensysteme zur Herstellung von pharmazeutischen Zubereitungen nach Anspruch 16, **dadurch gekennzeichnet**, daß man zur transdermalen Applikation hydrophiler Wirkstoffe ein wasserhaltiges offenes System herstellt.

**19.** Verwendung der Mehrkomponentensysteme zur Herstellung von pharmazeutischen Zubereitungen nach Anspruch 16, **dadurch gekennzeichnet**, daß man zur lokalen kutanen Applikation ein wasserhaltiges System herstellt.

## Claims

**1.** Transparent multi-component systems for pharmaceutical application containing one or several active ingredients in a system composed of an oil component, surfactants, co-surfactants and optionally water, characterised in that they contain up to 70% by wt. of the oil component, 2 to 60% by wt. surfactants, up to 78% by wt. co-surfactants and optionally up to 85% by wt. water, the sum of oil components and co-surfactants being at least 10% by wt., and in that they contain as surfactants physiologically compatible nonionic surfactants with an HLB value of more than 8 or amphoteric surfactants, and as co-surfactants partial esters and/or ethers of polyhydric alcohols which are liquid below 40 °C - except for those with less than three hydroxy groups - with saturated or unsaturated fatty acids and/or fatty alcohols with 6 to 22 C atoms or mixtures thereof, where optionally the co-surfactants can serve simultaneously as oil component in the system, or optionally the oil component can serve simultaneously as co-surfactant if oleyl alcohol and/or polar esters of monohydric alcohols with 2 to 4 C atoms with fatty acids with 8 to 18 C atoms are contained as oil component, systems containing isopropyl laurate and dodecanol simultaneously being excluded, and the individual components in the system are present in quantities such that liquid, single-phase systems which are stable at room temperature are formed which contain the active ingredient or ingredients in dissolved form.

**2.** Multi-component systems according to claim 1, characterised in that they contain as surfactants ethoxylated glycerin partial esters and/or ethers with aliphatic carboxylic acids or alcohols with 2 to 22 C atoms and with more than 7 to a maximum of 40 ethylene oxide units.

**3.** Multi-component systems according to claim 1, characterised in that they contain as surfactants polyethylene glycol esters and/or ethers with aliphatic carboxylic acids with 6 to 22 C atoms or with aliphatic alcohols with 6 to 22 C atoms.

**4.** Multi-component systems according to claim 1, characterised in that they contain as surfactants ethoxylated cyclopentanoperhydrophenanthrene derivatives with 5 to 50 ethylene oxide units.

**5.** Multi-component systems according to claim 1, characterised in that they contain as surfactants polyoxyethylene/polyoxypropylene copolymers.

**6.** Multi-component systems according to claim 1, characterised in that they contain as surfactants fatty acid-amidoalkyl betaines with ($C_2$ - $C_{22}$) fatty acids or lecithin.

**7.** Multi-component systems according to claims 1 to 6, characterised in that they contain as co-surfactants partial esters and/or ethers of polyhydric alcohols with saturated fatty acids and/or alcohols with 8 to 10 C atoms and mixtures thereof.

**8.** Multi-component systems according to claims 1 to 7, characterized in that they contain as co-

12

surfactants glycerin partial esters and/or ethers with fatty acids and/or fatty alcohols with 8 to 10 C atoms.

9.  Multi-component systems according to claims 1 to 8, characterised in that they contain as co-surfactants capric and/or caprylic acid glycerin esters, mixed capric-caprylic acid glycerin partial esters and mixtures thereof.

10. Multi-component systems according to claim 1, characterised in that they contain as surfactants PEG (20 EO) oleic acid glycerin partial esters and as co-surfactants capric-caprylic acid glycerin partial esters.

11. Multi-component systems according to claims 1 to 10, characterised in that they contain as oil component oleyl alcohol and/or polar esters of monohydric alcohols with 2 to 4 C atoms with fatty acids with 8 to 12 C atoms and as surfactants low molecular weight polyoxyethylene/polyoxypropylene copolymers with a PEG proportion of 20 to 50% by wt., based on the copolymer, and a molecular weight of the polypropylene proportion of 600 to 1400, where optionally the oil component in the system can serve simultaneously as co-surfactant.

12. Multi-component systems according to claims 1 to 11, characterised in that they contain additionally water and/or one or several hydrophilic components.

13. Multi-component systems according to claim 12, characterised in that they contain as hydrophilic component an aliphatic mono-or polyhydric $C_2$ to $C_4$ alcohol or urea alone or in combination or in combination with water.

14. Multi-component systems according to claims 1 to 13, characterised in that they contain 35 to 85% by wt. water.

15. Process for the preparation of the multi-component systems according to claims 1 to 14, characterised in that the surfactants and co-surfactants and optionally the oil component are mixed together, the pharmaceutical active ingredient is subsequently dissolved in the mixture, if necessary with heating, and optionally at the end the water is added at room temperature.

16. Use of the multi-component systems according to claims 1 to 14 for the preparation of pharmaceutical preparations for cutaneous, peroral, parenteral, vaginal or rectal administration and of inhalations, balneotherapeutics and cosmetic preparations.

17. Use of the multi-component systems for the preparation of pharmaceutical preparations according to claim 16, characterised in that a water-free occlusive system is prepared for transdermal application of lipophilic active ingredients.

18. Use of the multi-component systems for the preparation of pharmaceutical preparations according to claim 16, characterised in that a water-containing open system is prepared for transdermal application of hydrophilic active ingredients.

19. Use of the multi-component systems for the preparation of pharmaceutical preparations according to claim 16, characterised in that a water-containing system is prepared for local cutaneous application.

**Revendications**

1.  Systèmes à composants multiples transparents pour une utilisation pharmaceutique, qui contiennent un ou plusieurs additifs dans un système formé d'un composant d'huile, d'agents tensio-actifs, de co-agents tensio-actifs et, le cas échéant, d'eau, caractérisés en ce qu'ils contiennent jusqu'à 70% en poids du composant d'huile, 2 à 60% en poids des agents tensio-actifs, jusqu'à 78% en poids des co-agents tensio-actifs et, le cas échéant, jusqu'à 85% en poids d'eau, la somme des composants d'huile et des co-agents tensio-actifs étant d'au moins 10% en poids et en ce qu'ils comprennent, en tant qu'agents tensio-actifs, des agents tensio-actifs physiologiquement acceptables, non ioniques, ayant une valeur de HLB supérieure à 8 ou bien des agents tensio-actifs amphotères et, comme co-agents tensio-actifs, des esters et/ou éthers partiels liquides en dessous de 40°C d'alcools plurivalents, à l'exception de ceux ayant moins de trois groupes hydroxy, avec des acides gras saturés ou insaturés et/ou des alcools gras de 6 à 22 atomes de carbone ou bien un mélange de ceux-ci, ou,le ces échéant, les co-agents tensio-actifs peuvent servir simultanément de composant d'huile dans le système au bien le composant d'huile peut, le cas échéant, simultanément servir de co-agent tensio-actif, lorsque sont contenus en tant que composant d'huile, de l'alcool oléique et/ou un ester polaire d'alcools monovalents de 2 à 4 atomes de carbone avec des acides gras de 8 12 atomes de carbone, les systèmes contenant simultanément du laurate d'isopropyle et du dodécanol étant exclus,et les composants individuels dans le système étant présents en des quantités telles qu'il se forme,à la température ambiante, un système monophasé, stable et fluide, où est ou sont dissous le ou les additifs.

2. Systèmes à composants multiples selon la revendication 1, caractérisés en ce qu'ils contiennent, en tant qu'agents tensio-actifs,des esters partiels et/ou éthers de glycérine éthoxylées avec des acides carboxyliques aliphatiques ou, respectivement, des alcools de 2 à 22 atomes de carbone et avec plus de 7 jusqu'au maximum 40 unités d'oxyde d'éthylène.

3. Systèmes à composants multiples selon la revendication 1, caractérisés en ce qu'ils contiennent, en tant qu'agents tensio-actifs,un ester et/ou éther de polyéthylène glycol avec des acides carboxyliques aliphatiques de 6 à 22 atomes de carbone ou, respectivement, avec des alcools aliphatiques de 6 à 22 atomes de carbone.

4. Systèmes à composants multiples selon la revendication 1, caractérisés en ce qu'ils contiennent, en tant qu'agents tensio-actifs,des dérivés éthoxylés de cyclopentanoperhydrophénanthrène avec 5 à 50 unités d'oxyde d'éthylène.

5. Systèmes à composants multiples selon la revendication 1, caractérisés en ce qu'ils contiennent, en tant qu'agents tensio-actifs,des copolymérisats de polyoxyéthylène/polyoxypropylène.

6. Systèmes à composants multiples selon la revendication 1, caractérisés en ce qu'ils contiennent, en tant qu'agents tensio-actifs,une amido-alkyl-bétaïne d'acide gras avec des acides gras ($C_2$-$C_{22}$) ou bien de la lécithine.

7. Systèmes à composants multiples selon les revendications 1 à 6, caractérisés en ce qu'ils contiennent, en tant que co-agents tensio-actifs, des esters et/ou éthers partiels d'alcools plurivalents avec des acides gras saturés et/ou des alcools avec 8 à 10 atomes de carbone ainsi que leurs mélanges.

8. Systèmes à composants multiples selon les revendications 1 à 7, caractérisés en ce qu'ils contiennent,en tant que co-agents tensio-actifs, des esters et/ou éthers partiels de glycérine avec des acides gras et/ou des alcools gras de 8 à 10 atomes de carbone.

9. Systèmes à composants multiples selon les revendications 1 à 8, caractérisés en ce qu ils contiennent, en tant que co-agents tensio-actifs, un ester glycérique d'acide caprique et/ou caprylique, un ester glycérique partiel d'acides caprique-caprylique en mélange ainsi que des mélanges de ceux-ci.

10. Systèmes à composants multiples selon la revendication 1, caractérisés en ce qu ils contiennent, en tant qu'agents tensio-actifs, des esters glycériques partiels d'acide oléique PEG-(20 EO) et, en tant que co-agents tensio-actifs, des esters partiels glycériques d'acides caprique-caprylique.

11. Systèmes à composants multiples selon les revendications 1 à 10, caractérisés en ce qu'ils contiennent,en tant que composant d'huile, de l'alcool oléylique et/ou un ester polaire d'alcools monovalents de 2 à 4 atomes de carbone avec des acides gras de 8 à 12 atomes de carbone et,en tant qu'agents tensio-actifs, un copolymérisat de polyoxyéthylènepolyoxypropylène de faible poids moléculaire avec une proportion de PEG de 20-50% en poids, en se rapportant au copolymère, et un poids moléculaire de la partie de polypropylène de 600 à 1.400, le ces échéant, le composent d'huile pouvant servir simultanément,dans le système, de co-agent tensio-actif.

12. Systèmes à composants multiples selon les revendications 1 à 11, caractérisés en ce qu'ils contiennent de plus de l'eau et/ou un ou plusieurs composants hydrophiles.

14

**13.** Systèmes à composants multiples selon la revendication 12, caractérisés en ce qu'ils contiennent, en tant que composant hydrophile,un alcool aliphatique mono- ou plurivalent $C_2$ à $C_4$ ou bien de l'urée seule ou en combinaison ou bien en combinaison avec de l'eau.

**14.** Systèmes à composants multiples selon les revendications 1 à 13, caractérisés en ce qu'ils contiennent 35 à 85% en poids d'eau.

**15.** Procédé de production des systèmes à composants multiples selon les revendications 1 à 14, caractérisé en ce que l'on mélange les agents tensio-actifs et les co-agents tensio-actifs ainsi que, le cas échéant, le composent d'huile, ensuite on dissout l'additif pharmaceutique, le cas échéant en chauffant, dans le mélange et, le cas échéant, on ajoute à la fin de l'eau à température ambiante.

**16.** Utilisation des systèmes à composants multiples selon les revendications 1 à 14, pour la production de préparations pharmaceutiques pour application cutanée, per'os parentérale, vaginale ou rectale ainsi que d'inhalations, balnéothérapie ainsi que de préparations cosmétiques.

**17.** Utilisation des systèmes composants multiples pour la production de préparations pharmaceutiques selon la revendication 16,caractérisée en ce que l'on produit, pour application transdermique de l'additif lipophile, un système occlusif sans eau.

**18.** Utilisation du système à composants multiples pour la production de préparations pharmaceutiques selon la revendication 16, caractérisée en ce que l'on produit, pour une application transdermique de l'additif hydrophile, un système ouvert aqueux.

**19.** Utilisation des systèmes à composants multiples pour la production de préparations pharmaceutiques selon la revendication 16, caractérisée en ce que l'on produit, pour application locale cutanée, un système contenant de l'eau.

EP 0 152 945 B1

Figur 1: Serumspiegelkurven von tritiummarkiertem
Arecaidin-n-propylester-HCl im Blutserum von Kaninchen nach kutaner
Applikation gleicher Wirkstoffmengen in den Zubereitungen A bis E
(siehe Vergleichsbeispiel 1)

Figur 2: Drei-Komponentendiagramm, die schwarz
eingezeichneten Konzentrationsbereiche
stellen einphasige Bereiche dar

Figur 3: Plasmaspiegel von 5-Isosorbidmononitrat nach kutaner Applikation
beim Menschen (siehe Vergleichsbeispiel 2)

$\bigcirc$ 100 mg ISDN in einen hochdispersen System auf 150 $cm^2$ Haut

$\triangle$ 100 mg ISDN in einer Salbe auf 400 $cm^2$ Haut

EP 0 152 945 B1